# EUROPEAN PATENT APPLICATION

(11) **EP 1 358 850 A2**
(43) Date of publication of application: **05.11.2003**
(21) Application number: 03009046.8
(22) Date of filing: 17.04.2003
(51) Int. Cl.: A61B 17/12

(54) **Aneurysm treatment system**

(30) Priority: 25.04.2002 US 133180
(71) Applicant: Medtronic AVE Inc., Santa Rosa, CA 95403 (US)
(72) Inventor: Chu, Jack, Santa Rosa, CA 95409 (US); Raze, Brian, Windsor, CA 95492 (US)
(74) Representative: Hughes, Andrea Michelle

(57) **Abstract**

An aneurysm filling system includes a guide catheter 30, a delivery tubing 31 containing a plurality of embolizing units 15, 15a of an embolizing material, and a pushrod 35 within said delivery tube. The pushrod pushes the embolizing units out of the delivery tubing once the delivery tube has been guided through the guide catheter to a delivery position. The system is used for treating an aneurysm by deploying an endoluminal prosthesis and an embolizing material adjacent the aneurysm. The embolizing material is expanded to fill a portion of the aneurysm. The endoluminal prosthesis retains the expanded embolizing material within the aneurysm. A vascular implant system for treating an aneurysm includes an endoluminal prosthesis, a guide catheter including a delivery tubing slidably carried therein, and an embolizing material positioned within the deliver tubing. The embolizing material expands and fills a portion of the aneurysm when deployed from the delivery tubing. The prosthesis retains the expanded embolizing material within the aneurysm.

## Description

The present invention relates generally to the field of implantable medical devices. More particularly, the invention relates to an aneurysm treatment system.

Vascular aneurysms are produced when a thinning or weak spot in a vessel wall dilates eventually posing a health risk fiom its potential to rupture, clot, or dissect. While aneurysms can occur in any blood vessel, most occur in the aorta and peripheral arteries. The majority of aortic aneurysms occur in the abdominal aorta, usually beginning below the renal arteries and often extending into one or both of the iliac arteries. The etiology of aneuiysm formation is not entirely understood, but is thought to be related to congenital thinning of the artery, atherosclerotic vessel degeneration, vessel trauma, infection, smoking, high blood pressure, and other causes leading to vessel degeneration. Left untreated, aneurysms may lead to gradual vessel expansion, thrombus formation leading to stroke or other vessel blockage, vessel rupture, shock, and eventual death.

Aneurysms may be treated in open surgical procedures, where the diseased vessel segment is bypassed and repaired with an artificial vascular graft. While considered to be an effective surgical technique, particularly considering the alternative of the usually fatal ruptured aneurysm, conventional vascular graft surgery suffers fiom a number of disadvantages. The surgical procedure is complex and requires experienced surgeons and well equipped surgical facilities. Even with the best surgeons and equipment, patients suffering from such aneurysms are often elderly and weakened from cardiovascular and other diseases. This factor reduces the number of patients eligible for surgery. Even for eligible patients prior to rupture, conventional aneurysm repair has a relatively high mortality rate, usually from 2 to 10%. Morbidity related to the conventional surgery includes myocardial infarction, renal failure, impotence, paralysis, and other conditions. Even with successful surgery, recovery takes several weeks and often requires a lengthy hospital stay.

To overcome some of the drawbacks associated with open surgery, a variety of endovascular prosthesis placement techniques have been proposed. Without the need for open surgery, patient complications and recovery time may be significantly reduced. The most common type of aneurysm, the abdominal aortic aneurysm (AAA) may be used as an example for treatment with a prosthetic device. For example, one endovascular AAA repair technique involves a tubular prosthesis deployed by remote insertion through a femoral artery. The prosthesis may include a synthetic graft sheath body supported by an expandable stent. The stent may be self-expanding or balloon-expanding and typically includes means for anchoring the prosthesis to the vessel wall. The stent-graft prosthesis permits a shunt of blood flow from a healthy portion of the aorta, through the aneurysm, and into one or both of the iliac artery branches. The prosthesis excludes any thrombus present in the aneurysm while providing mechanical reinforcement of the weakened vessel reducing the risk of dissection and rupture, respectively.

One shortcoming associated with implanted endovascular prosthetics relates to migration and seal. The affected vessel(s) may vary widely in location, size, and the distended shape of the aneurysm itself. Particularly after treatment, the aneurysm and associated vessels may drastically change morphology thereby exerting stress forces on the deployed prosthesis. With sufficient change in aneurysm morphology and subsequent stress placed on the prosthesis, the device may migrate and/or detach from the vessel wall. As a result, the fluid seal may be compromised and blood may leak from the aorta into the aneurysm. The patient may have to undergo another treatment given the problem is detected early. The described and other undetected "endoleakage" may lead to aneurysm growth or regrowth, and to the more serious problems associated with aneurysms. Accordingly, it would be advantageous to minimize migration of the prosthesis and to maintain the fluid seal.

Another shortcoming associated with implanted endovascular prosthetics relates to healing response. The prosthesis provides an artificial structural support to the vessel region affected by the aneurysm. This may minimize the effect of blood pressure within the aneurismal sac, and reduce the chance of rupture. While the prosthetic provides benefits, it may not promote an optimal healing response within the aneurysm. To achieve a better healing response, a thrombus may be formed within the aneurismal sac. The thrombus, along with an implanted prosthesis, may occlude the aneurysm from vascular blood flow thereby optimizing the body's healing response. Accordingly, it would be desirable to provide a strategy for promoting thrombus formation in the aneurysm thereby aiding the healing response.

Therefore, it would be desirable to provide an aneurysm treatment system and method that overcomes the aforementioned and other disadvantages.

The invention provides an aneurysm filling system.
The system includes a guide catheter, a delivery tubing containing a plurality of embolizing units of an embolizing material, and a pushrod within said delivery tube. The pushrod pushes the embolizing units out of the delivery tubing once the delivery tube has been guided through the guide catheter to a delivery position. The embolizing unit may be operably attached to at least one other embolizing unit with a filamentous carrier. The embolizing material may be a hydrophflic foam material such as polyurethane, polyethylene, polyvinyl alcohol, HYPAN® hydrogel, styrene/polyvinyl-pyrolodone (PVP) copolymer, and polyacrylic acid copolymer. The embolizing material may be a hydrophobic foam material such as polyolefin, silicon, and vinyl acetate. The embolizing material may be thermoplastic and/or radiopaque. The embolizing material may include an open cellular structure and/or at least one therapeutic agent. An endoluminal prosthesis may be positioned within an aneurysm, wherein the endoluminal prosthesis retains the pushed embolizing units within the aneurysm. The endoluminal prosthesis may be a bifurcated stent-graft. The endoluminal prosthesis may be a self-expanding prosthesis or a balloon-expandable prosthesis.
endoluminal prosthesis may be a self-expanding prosthesis or a balloon-expandable prosthesis.

Another aspect according to the invention provides a vascular implant system for treating an aneurysm. The system includes means for deploying an endoluminal prosthesis and an embolizing material adjacent the aneurysm, and means for expanding the embolizing material to fill a portion of the aneurysm. The system further includes means for retaining the expanded embolizing material within the aneurysm with the endoluminal prosthesis.

Another aspect according to the present invention provides a vascular implant system for treating an aneurysm. The system includes an endoluminal prosthesis, a guide catheter including a delivery tubing slidably carried therein, and an embolizing material positioned within the delivery tubing. The embolizing material expands and fills a portion of the aneurysm when deployed from the delivery tubing. The prosthesis retains the expanded embolizing material within the aneurysm. The endoluminal prosthesis may include features described above. The embolizing material may include a plurality of embolizing units. The embolizing unit may be operably attached to at least one other embolizing unit with a filamentous carrier. The embolizing material may include features described above

Preferred embodiments will now be described by way of example only, with reference to the drawings.
FIGS. 1A and 1B are alternative embodiment perspective views of embolizing material formed into a plurality of embolizing units, in accordance with the present invention;
FIG. 2 is a cut-away view of a plurality of embolizing units positioned within a guide catheter, in accordance with the present invention;
FIGS. 3A and 3B are schematic views of an endoluminal prosthesis being deployed adjacent an abdominal aortic aneurysm by alternative methods;
FIG. 4 is a schematic view of embolizing material being deployed adjacent an abdominal aortic aneurysm and a deployed endoluminal prosthesis, in accordance with the present invention; and
FIG. 5 is a schematic view of a portion of a vascular implant system deployed for treating an aneurysm, in accordance with the present invention.

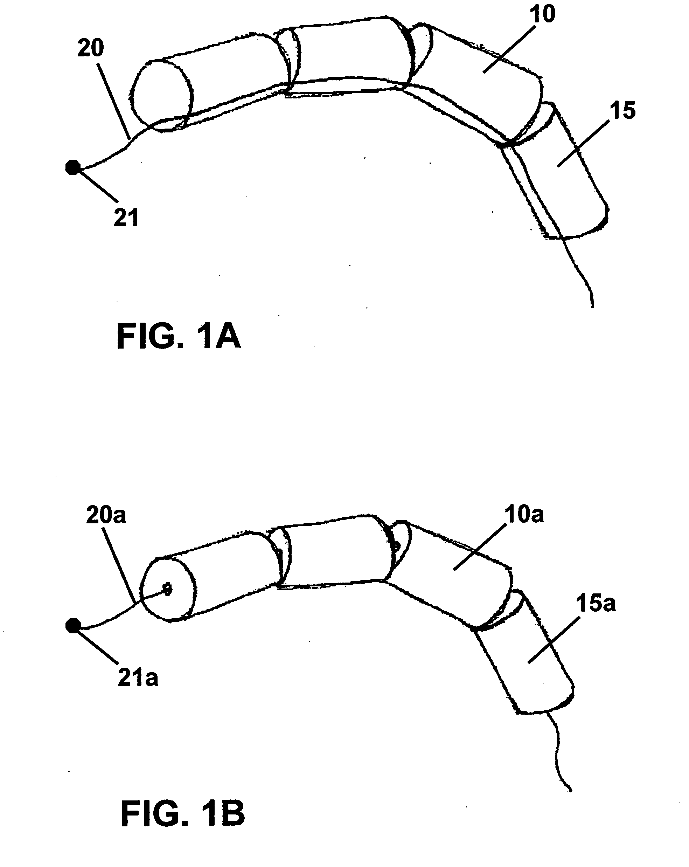

FIGS. 1A and 1B are alternative embodiment perspective views of embolizing material 10, 10a formed into a plurality of embolizing units 15, 15a made in accordance with the present invention. Embolizing material may be compressed before deployment within an aneurysm. Once in contact with a bodily fluid, such as blood, the embolizing material may become saturated and expand. Embolizing material may have an open cellular structure, spongiform in nature, thereby increasing surface area and fluid saturation rate. The increased clotting surface coupled with enhanced blood saturation may provide means for accelerating thrombus formation. The open cellular structure may be produced by foaming methods known in art (e.g., foaming agents, salts, etc.). The nature of the embolizing material and foaming method may influence the compressibility and expansion characteristics of the material.

In one embodiment, embolizing material may be a hydrophilic foam material such as polyurethane, , polyvinyl alcohol, HYPAN® hydrogel, styrene/polyvinyl-pyrolodone (PVP) copolymer, polyacrylic acid copolymer, and the like. Such hydrophilic foam materials may provide superior mechanical strength compared to other hydrophilic foam gels. As a result, they may be more resistant to creep, migration, fracture, and other shortcomings. In another embodiment, embolizing material may be a hydrophobic foam material such as polyolefin, polyethylene, polypropylene, silicone, and vinyl acetate. Such hydrophobic materials are generally biocompatible and have been routinely used in the manufacture of endovascular devices.

Embolizing material may include at least one therapeutic agent incorporated within and/or coated on its surface. The therapeutic agent may be a clotting factor (e.g., factors I-VIII, thrombin, fibrinogen), a tissue attachment factor (e.g., vitronectin, fibronectic, laminin, sclerosing agents: morrhuate sodium, ethanolamine oleate, tetradecyl sulfate), or other drug. The clotting factors and the open cellular structure of the embolizing material may accelerate thrombus formation, after their release into the aneurysm. The thiombus may occlude the aneurysm from vascular blood flow thereby optimizing the healing response. The tissue attachment factors may promote the incorporation of the embolizing material within the vessel tissue thereby enhancing its retention. A radiopaque material may be incorporated in the embolizing material, for example, when it is being melted. The radiopaque material may include barium sulfate, gold, silver, tantalum oxide, tantalum, platinum, platinum/iridium alloy, tungsten, and other materials used for imaging purposes.

Embolizing material may be thermoplastic thereby allowing melting and reshaping by extrusion, casting, thermal forming, and like processes. Embolizing material may be shaped and sized in a variety of geometries such as pellets, spheres, non-uniform shapes, or cylinders, as shown. The appropriate embolizing material shape and size may be determined by application and achieved by one of skill in the art.

In one embodiment, embolizing material 10, 10a may be shaped and sized into embolizing units 15, 15a to conform to a delivery catheter lumen. In another embodiment, embolizing material may be shaped and sized into embolizing units to conform to an aneurysm once expanded. For example, embolizing units may be shaped to a larger size to conform to a giant aneurysm. Those skilled in the art will recognize that the embolizing material may be formed in a variety of shapes other than the described embolizing units. In the following description, the embolizing unit is used as an exemplary form of the embolizing material.

Embolizing units 15, 15a may be operably attached to one another with a filamentous carrier 20, 20a. Filamentous carrier 20, 20a may be manufactured from steel, Nitinol, plastic, silk, wool, or other material providing sufficient tensile strength. Embolizing units 15, 15a may be variably spaced along filamentous carrier 20, 20a providing means for controlling amount of embolizing material 10, 10a for a given length. Filamentous carrier 20, 20a may include at least one attachment point 21, 21a for anchoring embolizing units to vessel wall and/or endoluminal prosthesis. Attachment point 21, 21a may include an adhesive or anchor member to secure embolizing material 10, 10a within aneurysm.

Filamentous carrier 20, 20a may be operably attached to embolizing units 15, 15a with various strategies. In one embodiment, as shown in FIG. 1A, filamentous carrier 20 may be operably attached to periphery of embolizing units 15 at spaced intervals. Filamentous carrier 20 may beattached to embolizing units 15 with sutures, adhesives, clips, and the like. In another embodiment, as shown in FIG. 1B, filamentous carrier 20a may be operably attached through a lumen formed in embolizing units 15a at spaced intervals. Those skilled in the art will recognize that the geometry, size, number, and attachment means of the embolizing units (e.g., 15), filamentous carrier (e.g., 20), and attachment point (e.g., 21) may vary without reducing the utility of the present invention.

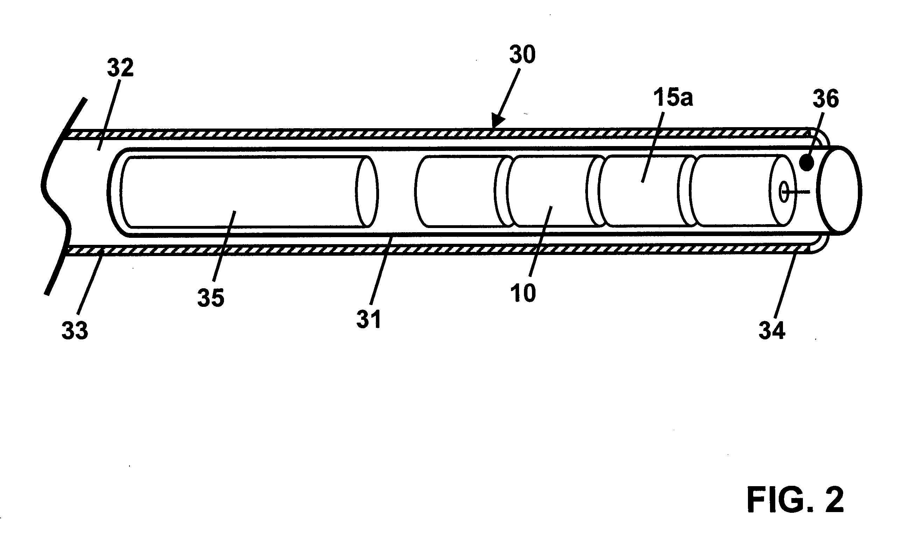

FIG. 2 is a cut-away view of a plurality of embolizing units 15a positioned within a guide catheter 30, in accordance with the present invention. Guide catheter 30 includes an elongated delivery tubing 31 slidably carried within a catheter lumen 32. Guide catheter 30 and delivery tubing 31 may be manufactured from a flexible material with high lubricity to minimize sliding friction between the guide catheter 30, delivery tubing 31, and embolizing units 15a. Adequate guide catheter 30 and delivery tubing 31 materials may include polytetrafluoroethylene (PTFE), high-density polyethylene (HDPE), and the like. The inside surface of the guide catheter 30 and delivery tubing 31 may be coated with a lubricity enhancing compound or coating, such as PhotoLink® lubricity coating made by SurModics, Inc., to further decrease the friction between the guide catheter 30, delivery tubing 31, and embolizing units 15a.

Lumen 32 may extend through guide catheter 30 axially from a proximal end 33 to a distal end 34 providing means for passage of delivery tubing 31 and embolizing units 15a to an aneurysm. Embolizing units 15a may be pre-loaded in delivery tubing 31 prior to deployment. Guide catheter 30 may include a pushrod 35 slidably position within delivery tubing 31 to deploy embolizing units 15. At least one marker 36 may be disposed on guide catheter 30 and/or delivery tubing 31 to allow in situ visualization. In one embodiment, marker 36 may be manufactured from a number of materials used for visualization in the art including radiopaque materials platinum, gold, tungsten, metal, metal alloy, and the like. Marker 36 may be visualized by fluoroscopy, IVUS, and other methods known in the art

Guide catheter 30, delivery tubing 31, and lumen 32 may vary in geometry and size to suit a given application. Additionally, embolizing units 15a material may be compressed to reduce the required size of the lumen 32, delivery tubing 31, and guide catheter 30. In one embodiment, delivery tubing 31 may have an outside diameter of about 0.6 to 4.5 mm for peripheral vascular applications. In another embodiment, lumen 32 may have a triangular, square, oval, round, or other cross-sectional shape to conform to embolizing units. Those skilled in the art will recognize that a wide variety of guide catheter 30 structures, including those capable of performing additional functions not described herein, may be readily adapted for use with the present invention. For example, guide catheter 30 may include a balloon coupled to inflation lumen and/or a delivery lumen with distal openings for substance delivery (e.g., therapeutic agents, contrast media, saline, fluids, and the like).

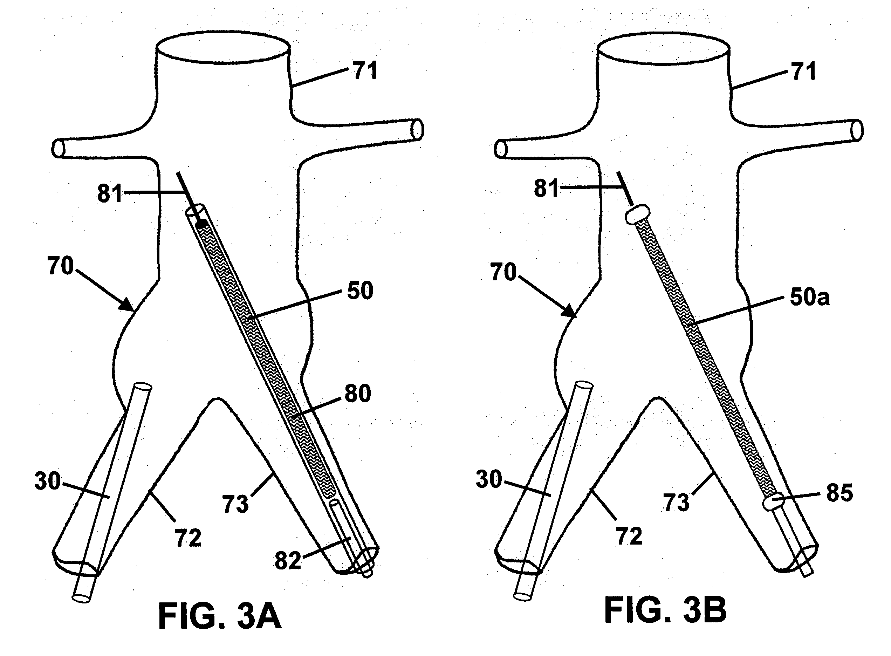

Referring now to FIGS. 3A and 3B schematic views made in accordance with the present invention are provided. An endoluminal prosthesis 50 is shown being deployed adjacent an abdominal aortic aneurysm 70 by alternative methods. Those skilled in the art will recognize that although the present invention is described primarily in the context of treating an abdominal aortic aneurysm, the inventors contemplate broader potential applicability. Any number of conditions compatible with intravascular embolization coupled with prosthesis deployment may benefit from the present invention, such as thoracic aortic or cranial aneurysms. Furthermore, the deployment of the endoluminal prosthetic assembly is not limited to the described strategy. Numerous modifications, substitutions, and variations may be made to the strategy while providing effective aneurysm treatment consistent with a configuration according to the present invention.

Treatment of the abdominal aortic aneurysm 70 includes deployment of the endoluminal prostheses 50, 50a. In one embodiment, as shown in FIG. 3A, a self-expanding endoluminal prosthesis 50 may be compressed within a flexible catheter 80 or other adequate delivery device as known in the art. In another embodiment, as shown in FIG. 3B, a balloon-expandable endoluminal prosthesis 50 may be compressed and disposed on a catheter-expandable balloon catheter 85 for deployment.

Aneurysm 70 treatment may begin by positioning a guide catheter 30 adjacent the aneurysm 70 via patient femoral artery and first iliac artery 72. The guide catheter 30 may be positioned during, after, or more preferably before the deployment of the endoluminal prosthesis 50. A guide wire 81 may then be positioned into the abdominal aorta 71 via patient femoral artery and second iliac artery 73. Catheter 80, 85 may then be advanced through a second iliac artery 73 and into abdominal aorta 71 using pre-positioned guide wire 81. It is important to note that pathways other than the described iliac arteries may be used to deploy the catheters 30, 80, and 85. In addition, the described deployment order may be varied during aneurysm 70 treatment.

Endoluminal prosthesis 50, 50a may then be positioned substantially within abdominal aorta 71 and second iliac artery 73 branch. Endoluminal prosthesis 50, 50a and guide catheter 30 position may be determined by visualization methods known in the art, such as fluoroscopy and/or intravascular ultrasound (IVUS). In one embodiment, radio-opaque markers disposed on portion of the endoluminal prosthesis 50, 50a and/or catheter 30, 80, and 85 may be visualized by fluoroscopy.

After appropriate positioning of guide 30 and endoluminal prosthesis delivery catheters 80, 85, endoluminal prosthesis 50, 50a may be deployed. As shown in FIG. 3A, a push rod 82 may be maintained in a fixed contact position with endoluminal prosthesis 50 as catheter 80 is withdrawn axially. Endoluminal prosthesis 50 may self-expand to a deployed diameter as catheter (catheter sheath) 80 is withdrawn. As shown in FIG. 3B, endoluminal prosthesis 50a may be balloon-expand to the deployed diameter as catheter-expandable balloon 85 is inflated. Endoluminal prosthesis 50, 50a deployed diameter may vary as required by application. A portion of the endoluminal prosthesis 50, 50a may be expanded into contact with abdominal aorta 71 during initial deployment.

As catheter 80 is further withdrawn, or catheter-expandable balloon catheter 85 is further inflated, first and second branch bodies of endoluminal prosthesis 50, 50a may be expanded into first iliac artery 72 and second iliac artery 73, respectively. Depending on the nature of the endoluminal prosthesis, the first or second branch body 51, 52 may be deployed in a separate step. This may be necessary when the endoluminal prosthesis 50, 50a, for example, has multiple pieces and requires in situ assembly. In one embodiment, the endoluminal prosthesis 50 may include a shortened branch (not shown), to which either branch body 51, 52 is attached. The branch body 51, 52 may be deployed with a catheter (not shown) through the appropriate iliac artery, and subsequently attached to the shortened branch. The branch body 51, 52 may seal to the shortened branch thereby extending the effective length of the endoluminal prosthesis 50 into the respective iliac artery 72, 73.

Endoluminal prosthesis 50, 50a maybe formed from a variety of materials used for expandable prosthetic devices known in the art. For example, endoluminal prosthesis 50, 50a may include covered stent design elements disclosed in U.S. Pat. No. 6,143,022 issued to Shull *et al*. Endoluminal prosthesis 50, 50a may further include pleated structure design elements disclosed in U.S. Pat. No. 5,607,464 issued to Trescony *et al*. In one embodiment, endoluminal prosthesis 50, 50a may be a stent-graft such as the AneuRx® device for endoluminal treatment. Those skilled in the art will recognize that endoluminal prosthesis 50, 50a geometry, size, and construction may vary without diminishing the utility of the present invention. In the presently described embodiment, the endoluminal prosthesis 50, 50a is a bifurcated stent-graft, however, tubular and branching prosthetic designs may be used.

Specifically, in one embodiment, endoluminal prosthesis 50, 50a may be formed from a plurality of support elements, such as a mesh of wires welded together at points of contact. Support elements may be manufactured from a resilient material known in the art, such as Nitinol, titanium, tantalum, stainless steel, metal alloy, polymer, and other biocompatible material capable of maintaining an expanded shape inside the vessel in which the device is deployed. Graft material may be disposed outside or inside of the support elements. Graft material may include any number of biocompatible, blood-impermeable graft membranes known in the art, such as polyester, polyethylene, polytetrafluoroethylene (PFTE), polyurethane, polypropylene, nylon, and the like. Graft material may be secured to support elements with a variety of strategies known in the art. Examples include suturing, adhesive bonding, heat welding, ultrasonic welding, and the like.

In one embodiment, first and second branch bodies may be expanded into contact with the wall of the aorta 71 and the second iliac artery 73. The leg portion of the endoluminal prosthesis that is positionable within the first iliac artery 72, is provided by means known to persons skilled in the art; e.g., by extending an everted leg from the interior of a one piece bifurcated prosthesis, by deploying a tubular prosthesis engagingly sealed in the lumen of the bifurcated prosthesis (for a two or more piece prosthesis) and extending to one or both respective iliac arteries, still further, the contralateral limb can be deployed by a delivery system that is inserted through the ipsilateral lumen, following a guidewire, bent around the iliac-aortic bifurcation and be deployed down from the main body of the endoluminal prosthesis (so that two catheters need not be present in one femoral-iliac artery at once). The endoluminal prosthesis 50, 50a-aorta 71 contact and the branch body-iliac artery 72, 73 contact may provide a fluid seal minimizing blood flow into aneurysm 70.

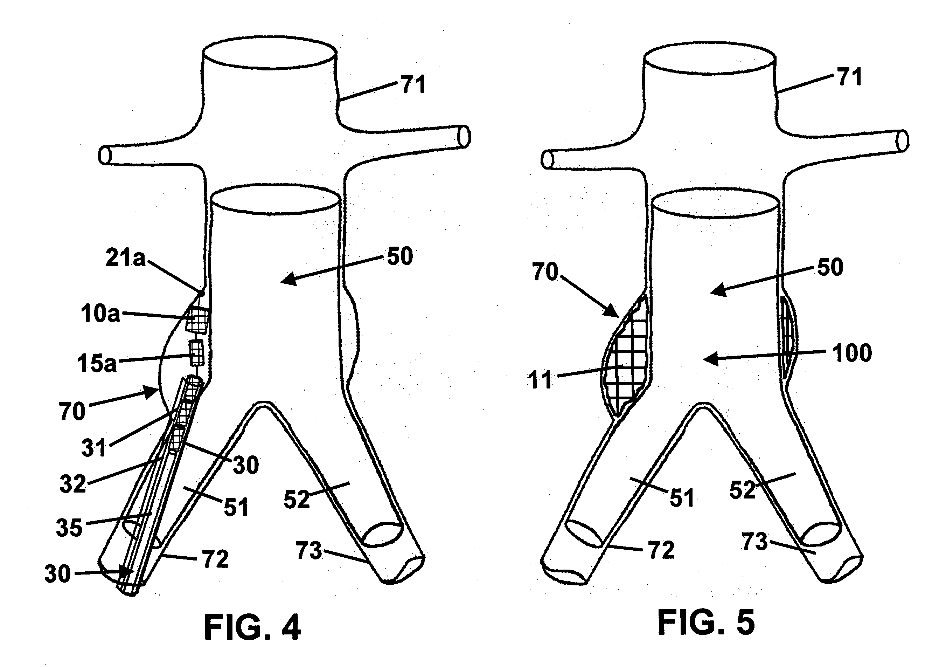

Catheter 80 and guide wire 81 may be removed from patient leaving guide catheter 30 positioned adjacent deployed endoluminal prosthesis 50, first branch body 51, and second branch body 52 as shown in FIG. 4. Embolizing units 15a may be deployed adjacent the aneurysm 70 and deployed endoluminal prosthesis 50 using the pre-positioned guide catheter 30. Embolizing units 15a may be deployed in the same or separate surgical procedure as the endoluminal prosthesis 50. Delivery tubing 31 may be advanced intravascularly, as previously described, through the guide catheter 30 (e.g., slidably advanced through guide catheter 30 lumen) until it is positioned adjacent aneurysm 70 and endoluminal prosthesis 50. Delivery tubing 31 may be positioned before, during, or (as shown) after the deployment of the endoluminal prosthesis 50.

Once delivery tubing 31 is positioned, and preferably after the endoluminal prosthesis 50 is deployed, the embolizing units 15a may be deployed. Pushrod 35 may be used to deploy the embolizing units 15a through delivery tubing 31 into aneurysm 70 space. The amount and/or number of embolizing units l5 deployed may be controlled by the length of the pushrod 35 forced into the delivery tubing 31. If necessary, additional embolizing units 15a may be deployed through lumen 32. In one embodiment, an empty delivery tubing may be slidably retrieved from guide catheter 30 and replaced with a delivery tubing 31 pre-loaded with additional embolizing units 15a. As such, embolizing units 15a may be repeatedly delivered to aneurysm 70 without having to substantially move the guide catheter 30. In another embodiment, additional embolizing units 15a may be added from delivery tubing 31 proximal end and subsequently push out from its distal end into the aneurysm 70.

During embolizing unit 15a deployment, attachment point 21a may be secured to aneurysm 70 wall and/or endoluminal prosthesis 50. Filamentous carrier 20a may link the embolizing units 15a together as one unit. The endoluminal prosthesis 50 provides a physical barrier preventing escape of the embolizing units 15a from the aneurysm 70. As such, the endoluminal prosthesis 50, attachment point 21a, and filamentous carrier 20a may each prevent migration of the embolizing units 15a by physically securing the embolizing material 10a within the aneurysm 70.

Visualization of the aneurysm 70 may be performed by methods known in the art to approximate its geometry and/or volume. A preliminary visualization may allow appropriate selection of embolizing unit geometry, size, and/or quantity that would expand to fill a desired portion of the aneurysm volume. Furthermore, visualization of radiopaque markers located in the embolizing units, guide catheter 30, delivery tubing 31 and/or endoluminal prosthesis 50 may provide means for monitoring aneurysm 70 treatment.

Embolizing units (e.g., 15) may absorb fluid (e.g., blood) from within the aneurysm 70 thereby filling the space as the embolizing material (e.g., 10) expands. The expansion may be accelerated by providing an additional fluid, such as a saline solution, through the guide catheter 30 to hydrate the aneurysm 70. Once the embolizing material expands, it may isolate and seal the aneurysm 70 from the blood supply augmenting any seal provided by the endolumi prosthesis 50. At least one therapeutic agent may be delivered as part of the embolizing units and/or through the guide catheter 30. The therapeutic agents may facilitate thrombus formation and enhance the retention of the embolizing units within the aneurysm.

After deployment of the embolizing units 15a, the guide catheter 30 maybe removed from aneurysm 70 site. A portion of a vascular implant system 100 for treating the aneurysm 70 may remain deployed, as shown in FIG. 5. The endoluminal prosthesis 50 and expanded embolizing material 11 of the vascular implant system 100 may fill a portion of the aneurysm 70. A thrombus may form within and/or around the expanded embolizing material 11. The endoluminal prosthesis 50 and thrombus may seal the aneurysm from vascular blood flow minimizing "endoleakage" and optimizing the body's healing response. The thrombus and expanded embolizing material 11 may provide mechanical support to the endoluminal prosthesis 50 thereby further minimizing migration and "endoleakage". The endoluminal prosthesis 50 may retain the expanded embolizing material 11 and the thrombus within the aneurysm 70. This may minimize the risk of an embolus migrating from the aneurysm producing deleterious effects, such as stroke, elsewhere in the body.

## Claims

1. An aneurysm filling system comprising:
a guide catheter (30);
a delivery tubing (31) containing a plurality of embolizing units (15,15a) of an embolizing material;
a pushrod (35) within said delivery tube to push the embolizing units out of the delivery tubing once the delivery tube has been guided through the guide catheter to a delivery position.

2. The system of claim 1 wherein the embolizing units (15,15a) are operably attached to at least one other embolizing unit with a filamentous carrier.

3. The system of claim 1 or 2 wherein the embolizing material comprises a hydrophilic foam material.

4. The system of claim 3 wherein the hydrophilic foam material is selected from a group consisting of polyurethane, polyvinyl alcohol, HYPAN® hydrogel, styrene/polyvinyl-pyrolodone (PVP) copolymer, and polyacrylic acid copolymer.

5. The system of claim 1 or 2 wherein the embolizing material comprises a hydrophobic foam material.

6. The system of claim 5 wherein the hydrophobic foam material is selected form a group consisting of polyolefin, silicon, polyethylene and vinyl acetate.

7. The system of claim 1 or 2 wherein the embolizing material is thermoplastic.

8. The system of any preceding claim wherein the embolizing material is radiopaque.

9. The system of claim 1 or 2 wherein the embolizing material comprises an open cellular structure.

10. The system of any preceding claim wherein the embolizing material comprises at least one therapeutic agent.

11. The system of any preceding claim further comprising an endoluminal prosthesis (50,5a) positioned within an aneurysm (70), wherein the endoluminal prosthesis retains the pushed embolizing units within the aneurysm.

12. The system of claim 11 wherein the endoluminal prosthesis comprises a bifurcated stent-graft.

13. The system of claim 11 wherein the endoluminal prosthesis comprises a self-expanding prosthesis.

14. The system of claim 11 wherein the endoluminal prosthesis comprises a balloon-expandable prosthesis.

15. A vascular implant system for treating an aneurysm, comprising;
means (30) for deploying an endoluminal prosthesis and an embolizing material adjacent the aneurysm;
means (80) for expanding the embolizing material to fill a portion of the aneurysm; and
means for retaining the expanded embolizing material within the aneurysm with the endoluminal prosthesis.

16. A vascular implant system as claimed in claim 15 wherein said means (30) for deploying, comprises a guide catheter including a delivery tubing slidably carried therein; and further comprising and embolizing material positioned within the delivery tubing, wherein the embolizing material expands and fills a portion of the aneurysm when deployed from the delivery tubing; and further comprising an endoluminal prosthesis (50,5a) which retains the expanded embolizing material within the aneurysm.

17. The system of claim 16 wherein the endoluminal prosthesis comprises a bifurcated stent-graft.

18. The system of claim 16 wherein the endoluminal prosthesis comprises a self-expanding prosthesis.

19. The system of claim 16 wherein the endoluminal prosthesis comprises a balloon-expandable prosthesis.

20. The system of any of claims 16 to 19 wherein the embolizing material comprises a plurality of embolizing units.

21. The system of claim 20 wherein the embolizing units are operably attached to at least one other embolizing unit with a filamentous carrier.

22. The system of any of claims 16 to 21 wherein the embolizing material comprises a hydrophilic foam material.

23. The system of claim 22 wherein the hydrophilic foam material is selected from a group consisting of polyurethane, polyvinyl alcohol, HYPAN® hydrogel, styrene/polyvinyl-pyrolodone (PVP) copolymer, and polyacrylic acid copolymer.

24. The system of any of claims 16 to 21 wherein the embolizing material comprises a hydrophobic foam material.

25. The system of claim 24 wherein the hydrophobic foam material is selected from a group consisting of polyolefin, silicon, polyethylene and vinyl acetate.

26. The system of claim 16 wherein the embolizing material is thermoplastic.

27. The system of claim 16 wherein the embolizing material is radiopaque.

28. The system of claim 16 wherein the embolizing material comprises an open cellular structure.

29. The system of claim 16 wherein the embolizing material comprises at least one therapeutic agent.
